# EUROPEAN PATENT APPLICATION

(11) **EP 3 025 641 A1**
(43) Date of publication of application: **01.06.2016**
(21) Application number: 15193118.5
(22) Date of filing: 05.11.2015
(51) Int. Cl.: A61B 5/00

(54) **PHOTOACOUSTIC DEVICE AND CONTROL METHOD FOR PHOTOACOUSTIC DEVICE**

(30) Priority: 28.11.2014 JP 2014241830
(71) Applicant: Canon Kabushiki Kaisha, Tokyo, 146-8501 (JP)
(72) Inventor: NAKAJIMA, Takao, Tokyo, Tokyo 146-8501 (JP); TOKITA, Toshinobu, Tokyo, Tokyo 146-8501 (JP)
(74) Representative: TBK

(57) **Abstract**

A photoacoustic device comprises a light irradiation unit configured to irradiate a subject with light; an acoustic wave detector configured to detect an acoustic wave generated inside the subject and convert the detected acoustic wave into an electric signal; a first photodetector configured to acquire information relating to an intensity of light that has propagated inside the subject and leaked to the outside of the subject; an information acquisition unit configured to acquire information on an interior of the subject on the basis of the electric signal; and a control unit configured to control the light with which the subject is irradiated on the basis of the information relating to the intensity of light which has been acquired by the first photodetector.

## Description

### BACKGROUND OF THE INVENTION

### Field of the Invention

The present invention relates to a photoacoustic device for measuring information on the interior of a subject by using light.

### Description of the Related Art

In recent years, research aimed at imaging morphological information and physiological information, that is, functional information, on the interior of a subject has been advanced in the medical field. Photoacoustic imaging has recently been suggested as one of such techniques.

Where a living body is irradiated as a subject with light such as pulsed laser light, an acoustic wave (typically an ultrasound wave) is generated when the light is absorbed by the biological tissue inside the subject. This effect is called a photoacoustic effect, and the acoustic wave generated by the photoacoustic effect is called a photoacoustic wave. Since the tissues constituting the subject differ in the absorbance of light energy, the sound pressure of the generated photoacoustic wave also differs. In the photoacoustic imaging, by receiving the generated photoacoustic wave with a probe and mathematically analyzing the received signals, it is possible to image the optical properties, in particular the distribution of light absorption coefficient, inside the subject.

Further, on the basis of the obtained light absorption coefficient distribution, it is possible to determine the content ratio of oxyhemoglobin in the total hemoglobin in blood, that is, oxygen saturation. Since the oxygen saturation is an indicator for distinguishing between benign and malignant tumors, it is expected to be an efficient means for revealing malignancies.

By combining those indicators it is possible to acquire both the morphological information (for example, vasculature) and functional information (for example, oxygen saturation) in the interior of the subject.

The possibility of increasing the resolution with the object of imaging light-absorbing bodies with better accuracy has also been studied in recent years. For example, Japanese Translation of PCT Application No. 2011-519281 discloses a technique for increasing the resolution by focusing irradiation light with a lens and disposing a subject at the focal position of the light. As a result, the absorbing bodies such as fine blood vessels in the vicinity of the subject surface can be imaged with a high resolution.

### SUMMARY OF THE INVENTION

By focusing the irradiation light as described in Japanese Translation of PCT Application No. 2011-519281, it is possible to narrow down the region where the acoustic wave is generated and increase the imaging resolution. However, when the subject is a living body, the subject can be damaged when the intensity of the irradiation light is too high, and therefore limitless increase in light quantity is impossible. In particular, the quantity of light with which a human body can be irradiated is stipulated as maximum permissible exposure (MPE) by American National Standards Institute (ANSI) or Japanese Industrial Standard (JIS). Therefore, the intensity of the irradiation light emitted from a photoacoustic device needs to be set within a range in which the MPE for a human body is not exceeded. For example, when the skin is irradiated with light, the irradiation light intensity needs to be equal to or less than the MPE which has been set for the skin.

Meanwhile, since the irradiation light emitted from a photoacoustic device is diffused inside the subject, where the measurements are performed on a human face, or the like, by using the photoacoustic device, even when the irradiation is performed with light at or below the MPE for the skin, the irradiation light can diffuse inside the living body and the propagating light can reach cornea or retina. Further, part of the irradiation light diffused inside the subject can be emitted to the outside of the subject and the emitted light can fall again on the eyes as a result of reflection from reflecting materials, or the like.

Further, a different value of MPE is stipulated for each segment to be irradiated with light. For example, the MPE for the eyes (cornea and retina) is less than the MPE for the skin. Since the MPE for a human body thus differs depending on the segment, in some cases, sufficient safety cannot be ensured by taking into account only the MPE of the irradiation portion.

The present invention was devised in view of the foregoing problems.

The present invention in its first aspect provides a photoacoustic device as specified in claims 1 to 13.

The present invention in its second aspect provides a control method for a photoacoustic device as specified in claim 14.

The present invention in its third aspect provides a non-transitory computer readable storing medium as specified in claim 15.

In accordance with the present invention, safety of a photoacoustic device can be increased.

Further features of the present invention will become apparent from the following description of exemplary embodiments with reference to the attached drawings.

### BRIEF DESCRIPTION OF THE DRAWINGS

FIG. 1 illustrates the configuration of a photoacoustic measurement device according to the first embodiment;
FIG. 2 is a measurement flowchart in the first embodiment;
FIG. 3 is a measurement flowchart in the first embodiment;
FIG. 4 is a measurement flowchart in the second embodiment; and

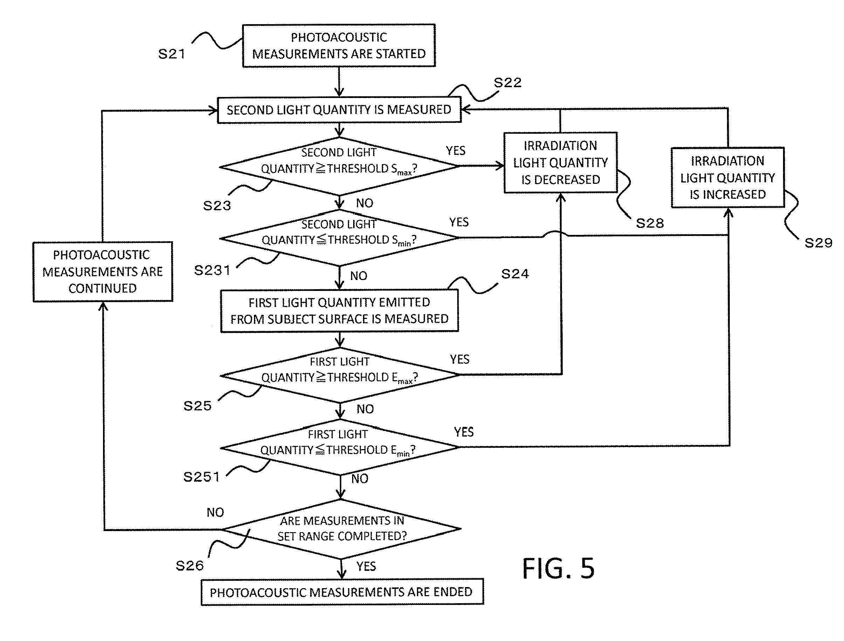

FIG. 5 is a measurement flowchart in the third embodiment.

### DESCRIPTION OF THE EMBODIMENTS

Embodiments of the present invention will be described hereinbelow in detail with reference to the drawings. Like constituent elements are, in principle, assigned with like reference numerals and explanation thereof is omitted. Numerical values and materials used in the explanation of embodiments place no limitation on the scope of the invention.

### (First Embodiment)

In the photoacoustic measurement device according to the first embodiment, a subject is irradiated with pulsed light, and a photoacoustic wave generated in the subject under the effect of the pulsed light is received and analyzed, thereby visualizing, that is, imaging information relating to optical properties inside the subject (referred to hereinbelow as "property information"). The property information, as referred to herein, is information relating, for example, to absorbed amount and absorption rate of optical energy, more specifically, property values relating to an absorption coefficient and concentration of the substance constituting the tissue. The information relating to concentration density of the substance, as referred to herein, is for example, oxygen saturation, total hemoglobin concentration, and oxyhemoglobin or deoxyhemoglobin concentration. It may also be glucose concentration, collagen concentration, melanin concentration, and volume fractions of fats and water. Those types of information are generated as two-dimensional or three-dimensional distribution data and outputted as images.

The main objective of the photoacoustic measurement device according to the present embodiment is to perform diagnostic of vascular diseases and malignant tumors in humans and animals and follow-up observations after chemical treatment. Therefore, an examination object such as part of a living body, more specifically skin and subcutaneous segments of humans and animals, is assumed as the subject. The device can be used particularly advantageously when a shallow segment within several millimeters from the skin surface and also blood vessels in the dermis layer are the examination objects.

### <System Configuration>

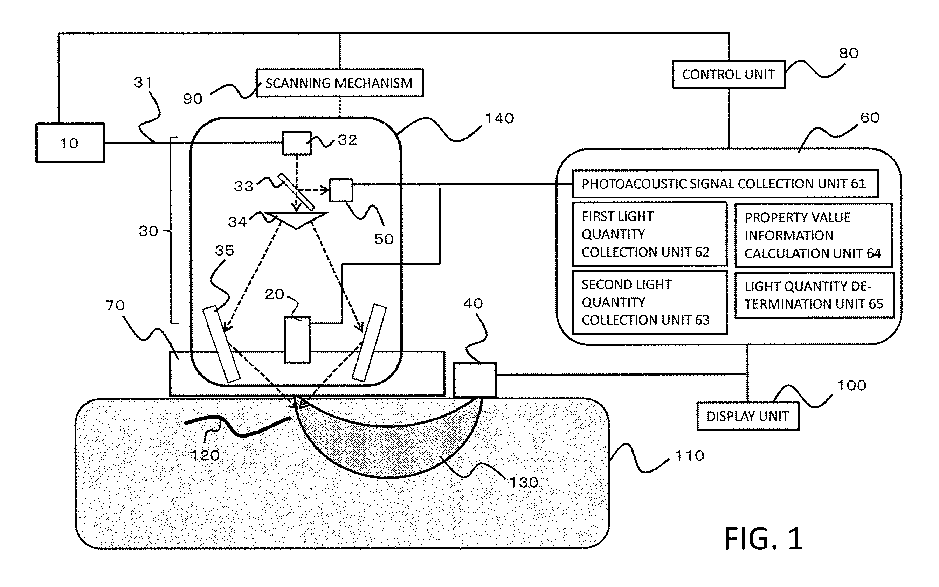

The configuration of the biophotonic measurement device according to the first embodiment will be explained hereinbelow with reference to FIG. 1.

The biophotonic measurement device according to the first embodiment is constituted by a light source 10, a probe 20, an optical system 30 (represented in the figures by reference numerals 31 to 35), a first photodetector 40, a second photodetector 50, a processing unit 60, an acoustic matching member 70, a control unit 80, a scanning mechanism 90, and a display unit 100.

The constituent elements are described hereinbelow.

### «Light Source 10»

The light source 10 is a unit (light irradiation unit) that generates pulsed light and irradiates the subject therewith.

In order to obtain large power, it is desirable that the light source be a laser source, but a light-emitting diode, a flash lamp or the like can be also used instead of the laser. When a laser is used as the light source, a solid-state laser, gas laser, dye laser, or semiconductor laser can be used.

Ideally, a Nd:YAG-excitation OPO laser, dye laser, Ti:sa laser, or alexandrite laser which have a high output and a continuously variable wavelength may be used. Further, a plurality of single-wavelength lasers of different wavelengths may be used.

It is desirable that the wavelength of pulsed light be a wavelength ensuring the propagation of light to the interior of the subject, that is, a specific wavelength absorbable by a specific component among the components constituting the subject. More specifically, where the subject is a living body, the desirable wavelength is 400 nm (inclusive) to 1600 nm (inclusive). In particular, when blood vessels close to the living body surface are to be imaged with a high resolution, the desirable wavelength is 400 nm (inclusive) to 700 nm (inclusive). Further, when deep regions of a living body are imaged, a wavelength with low absorption in the background tissue of the living body (700 nm (inclusive) to 1100 nm (inclusive)) is preferred. Terahertz wave, microwave, and radio wave regions can be also used.

In order to generate the photoacoustic wave effectively, it is necessary that light irradiation be performed for a sufficiently short time corresponding to the thermal properties of the subject. When the subject is a living body, it is preferred that the pulse width of the pulsed light generated from the light source be from about 1 nanosec to about 100 nanosec.

When light of a plurality of wavelengths is used for measurements, it is more preferred that a laser with a variable wavelength of light generation be used. Further, when the subject is irradiated with the light of a plurality of wavelengths, the irradiation may be performed while switching a plurality of lasers generating light beams of mutually different wavelengths.

The pulsed light generated from the light source is referred to hereinbelow as irradiation light. The irradiation light emitted from the light source is used for irradiating the subject through the below-described optical system.

### «Probe 20»

The probe 20 is a unit that receives the acoustic wave generated inside the subject and converts the received wave into an electric signal. The probe is an assembly of a plurality of acoustic elements. The acoustic wave receiving unit is also referred to as an acoustic wave detector and transducer. The acoustic wave, as referred to in the present invention, is typically an ultrasound wave and is inclusive of elastic waves referred to as sound waves, ultrasound waves, photoacoustic waves, and photo-ultrasound waves.

The acoustic wave generated from a living body is an ultrasound wave with a frequency from 100 KHz to 100 MHz. Therefore, acoustic elements capable of receiving this frequency band are used for the probe 20. More specifically, a transducer using a piezoelectric effect, a transducer using light resonance, and a transducer using a change in capacity can be used.

Further, it is desirable that the probe 20 be used that has a high sensitivity and a broad frequency band. More specifically, a PZT (piezoelectric ceramics), PVDF (polyvinylidene fluoride resin), CMUT (capacitative micromachine ultrasound transducer), and Fabry-Perot interferometer can be used. However, the probes listed herein are not limiting, and any device may be used, provided that it can function as a probe.

The probe 20 may be also constituted by an array of a plurality of acoustic elements. The array is preferably an arrangement in which acoustic elements are arranged side by side on a flat or curved surface, such as called 1D array, 1.5D array, 1.75D array, and 2D array. By receiving acoustic waves at the same time at a plurality of positions, it is possible to shorten the measurement time and reduce the effect of vibrations of the subject, or the like.

The probe 20 may be a focus-type probe incorporating an acoustic lens and may also incorporate an amplifier for signal amplification.

### <<Optical System 30>>

The optical system 30 is an optical member that guides the light generated by the light source 10 to the surface of the subject.

The optical system typically guides the irradiation light to a subject while processing the light into the desired irradiation light distribution shape by using optical components such as a waveguide, such as optical fibers, a mirror that reflects the light, a lens that expands the light, and a diffusion plate that diffuses the light. Such optical components may be of any types, provided that the subject can be irradiated in the desired form with the irradiation light emitted from the light source. In particular, the imaging resolution can be increased by irradiating the subjects with a focused light beam.

In the present embodiment, the combination of a waveguide 31 (typically, an optical fiber), a collimator lens 32, a beam splitter 33, an axicon lens 34, and a mirror 35 is referred to as the optical system 30. By using such a configuration, it is possible to focus the irradiation light at the target position of the subject.

It is preferred that the beam splitter 33 be used such that the reflectance at an incidence angle of 45 degrees be within 5%.

Further, the optical system 30 is not necessarily of type such that the subject is irradiated with the focused irradiation light. For example, in an apparatus designed to examine infants, it is sometimes preferred that the subject be irradiated with an expanded light beam.

### «First Photodetector 40 and Second Photodetector 50»

The first photodetector 40 is a unit configured to detect light that has leaked to the outside of the subject, of the light with which the subject 11 has been irradiated and which has diffused and propagated inside the subject, and measure the quantity (that is intensity) of this light. The light that has reached the first photodetector passes through a variety of paths while diffusing inside the subject. The range of the propagation paths of this light has a banana-like shape such as denoted by the reference numeral 130.

For example, a photodiode, an avalanche photodiode, and a photoelectronic multiplier can be used in the first photodetector 40, but other devices can be also used, provided that light can be detected. For example, a light energy meter can be also used.

It is preferred that the first photodetector 40 be in intimate contact with the surface of the subject 11. In particular, when the subject 11 is a human body, it is preferred that the first photodetector be in intimate contact with the skin.

The second photodetector 50 detects part of the irradiation light guided by the beam splitter 33 and measures the quantity of this light. A photodiode or a light energy meter can be advantageously used for the second photodetector 50, but other devices may be also used.

### <<Processing Unit 60>>

The configuration of the processing unit 60 is explained hereinbelow. In the present embodiment the processing unit 60 is constituted by a photoacoustic signal collection unit 61, a first light quantity collection unit 62, a second light quantity collection unit 63, a property value information calculation unit 64, and a light quantity determination unit 65. Each of those units will be explained below.

The photoacoustic signal collection unit 61 performs the processing of collecting time-series analog signals, which are outputted from the acoustic elements of the probe 20, for each channel, amplifying and A/D converting the signals, and temporarily storing the digitalized signals. The photoacoustic signal collection unit 61 typically uses a circuit called data acquisition system (DAS).

The first light quantity collection unit 62 collects the signals outputted form the first photodetector 40, and the second light quantity collection unit 63 collects the signals outputted from the second photodetector 50. The first light quantity collection unit 62 and the second light quantity collection unit 63 perform, as necessary, the processing of amplifying the signals, A/D converting the analog signals, temporarily storing the digitalized signals, and converting the acquired signals into light quantity values. The quantity of light incident on each photodetector can thus be acquired.

The first light quantity collection unit 62 and the second light quantity collection unit 63 have, for example, an amplifier that amplifies the signals, an A/D converter that digitalizes the analog signals, and an operational unit that converts the signals into light quantity values.

The first light quantity collection unit 62 is preferably configured to acquire the quantity of light (per unit surface area), which is incident on the first photodetector 40, on the basis of the signals outputted from the first photodetector 40. Therefore, it is preferred that the first light quantity collection unit 62 stores a coefficient or formula necessary for the conversion.

Further, the second light quantity collection unit 63 is preferably configured to acquire the quantity of light (per unit surface area), which is irradiated onto the subject 11, on the basis of the signals outputted from the second photodetector 50. Therefore, it is preferred that the second light quantity collection unit 63 stores a coefficient or formula necessary for the conversion.

The property value information calculation unit 64 is a unit (information acquisition unit) that uses the signals outputted from the photoacoustic signal collection unit 61 and the first light quantity collection unit 62 to calculate property information (that is, light absorbance, and the like) on the interior of the subject for each position inside the subject. The property value information relating to light absorbance at each position inside the subject is referred to hereinbelow as light absorption distribution inside the subject.

The photoacoustic measurement device according to the present embodiment can be also implemented as a photoacoustic microscope or a photoacoustic tomography device.

When the device is a photoacoustic microscope, the property value information calculation unit 64 performs envelope detection of the signal outputted from the photoacoustic signal collection unit 61 with respect to time changes, then converts the time axis direction in the signal of each pulse into depth direction, and plots on spatial coordinates. Sound pressure distribution data are acquired by performing such processing for each measurement position (scanning position). Further, the property value information calculation unit 64 acquires the light absorption distribution data for each measurement position by using the light quantity values obtained with the first light quantity collection unit 62 and correcting the sound pressure distribution at each measurement position. For example, when the first photodetector 40 is a photodiode, the light absorption distribution area for each measurement position can be acquired by taking the peak value of the signal outputted from the photodiode in each measurement point and dividing the sound pressure distribution data by the peak value.

Meanwhile, when the device is a photoacoustic tomography device, the property value information calculation unit 64 finds initial sound pressure data corresponding to a position on two-dimensional or three-dimensional spatial coordinates by using the reception signal for each channel which is outputted from the photoacoustic signal collection unit 61 and performing image reconstruction. The property value information calculation unit 64 can use a well-known reconstruction method such as a UBP method or FBP method for image reconstruction. Further, the property value information calculation unit 64 may use phasing addition processing for image reconstruction. The property value information calculation unit 64 can also acquire the light absorption distribution data for each measurement position by correcting the sound pressure distribution data by using light quantity values outputted from the first light quantity collection unit 62.

Further, with measurements using a plurality of wavelengths, the property value information calculation unit 64 may determine the concentration distribution of a substance present inside the subject by using light absorption distribution data for each wavelength. In particular, by using the concentration of oxyhemoglobin (HbO) and the concentration of deoxyhemoglobin (Hb), it is possible to determine the oxygen saturation distribution in blood.

The light quantity determination unit 65 determines the relation between the light quantity values outputted from the first light quantity collection unit 62 and the second light quantity collection unit 63 and preset thresholds and transmits the determination result to the control unit 80. A specific example of such processing is described below.

The property value information calculation unit 64 and the light quantity determination unit 65 can be configured of a processor such as a CPU or GPU and an operational circuit such as a FPGA chip. Those units can be configured not only of one processor or operational circuit, but also of a plurality of processors or operational units. A memory that stores the reception signals, generated distribution data, display image data, and various measurement parameters may be also provided. The memory is typically constituted by one or more memory media such as ROM, RAM, and hard disk.

### «Acoustic Matching Member 70»

The acoustic matching member 70 is a container in which an acoustic matching material is accommodated. Typically it is a water tank or water bag. In the present embodiment, the acoustic element of the probe 20 is configured to be immersed in the acoustic matching material. As a result, acoustic impedance matching can be performed between the subject 11 and the acoustic element. It is also preferred that the surface of the acoustic matching member 70 which is in contact with the subject 11 be constituted by a film with a thickness less than the wavelength of the photoacoustic wave thereby facilitating the passage of the photoacoustic wave. More preferably the contact surface has a thickness equal to or less than a quarter of the wavelength of the photoacoustic wave. It is also preferred that the acoustic matching material and contact surface be configured of a material that hardly absorbs the irradiation light. For example, water and ultrasound gel or oil can be used as the acoustic matching material, and polyethylene, or the like, can be used as the material of the contact surface.

It is also preferred that the acoustic matching between the contact surface and the subject 11 be performed with a gel, or the like.

### <<Control Unit 80>>

The control unit 80 controls the operation of each of the above-described units. For example, the control unit supplies a signal indicating light emission or a signal setting the intensity of light to the light source 10, supplies a signal that controls the reception of the acoustic wave to the probe 20, and supplies a signal that controls the position to the below-described scanning mechanism 90. Further, the control unit 80 has a function of controlling the emission or quantity of the irradiation light on the basis of the determination result obtained by the light quantity determination unit 65. The control unit also performs signal amplitude control of the processing unit 60, A/D conversion timing control, and storage control of the reception signals.

Similarly to the processing unit 60, the control unit 80 can be also configured of a combination of a processor such as a CPU and GPU and a circuit such as a FPGA chip, or by a combination of a plurality thereof. A memory for storing various measurement parameters may be also provided. The memory is typically configured of one or more memory media such as a ROM, RAM, and hard disk. Those components can be also used together with the processing unit 60.

### «Scanning Unit 90»

The scanning mechanism 90 moves the probe 20 and the optical system 30 with respect to the subject. In the present embodiment, both the probe 20 and the optical system 30 are moved synchronously by the scanning mechanism 90. A combination of the probe 20 and the optical system 30 is referred to hereinbelow as a measurement unit 140.

The scanning mechanism 90 is typically a stage provided with a stepping motor or servo motor, but other configurations may be also used. By moving the measurement unit 140, the scanning mechanism 90 enables measurements at a plurality of measurement positions on the subject 110.

In the present embodiment, the probe 20 and the optical system 30 are operated at the same time, but a configuration may be also used in which, for example, the optical system 30 is fixed and only the probe 20 is scanned while irradiating a wide range on the subject 110 with the irradiation light.

It is also possible to fix the probe 20 and scan only the optical system 30. More specifically, a probe capable of receiving photoacoustic waves from a wide range, such as a single transducer or array-type transducer with a wide focus range, is used as the probe 20 and the probe 20 is fixed. Further, the subject is irradiated with the focused irradiation light which can be scanned with the scanning mechanism 90.

Where the probe 20 is thus fixed, it is not necessary to use a liquid such as water for acoustic matching. For example, a gel member (polyurethane gel or the like) can be used instead of the acoustic matching member 70.

Where the scanning mechanism 90 changes the irradiation position of the irradiation light or the acquisition position of the acoustic wave, it is possible not to move the probe 20 or the optical system 30 itself. For example, a mirror for guiding the acoustic wave or irradiation light may be provided and the position may be changed by moving the mirror. For example, the mirror angle may be changed or a mirror position may be changed. A galvano mirror or MEMS mirror can be used as such mirror.

In this case, it is also possible to move both the irradiation position of the irradiation light and the acquisition position of the acoustic wave, or only one of them.

### «Display Unit 100»

The display unit 100 displays images and is typically a liquid crystal display, CRT, or organic EL display. The display unit 100 is not necessarily a part of the photoacoustic measurement device according to the present embodiment and may be connected externally.

### «Subject 110»

The subject 110 is explained hereinbelow, although it is not a component constituting the photoacoustic measurement device according to the present embodiment. The main objective of the photoacoustic measurement device according to the present embodiment is to perform diagnostic of vascular diseases and malignant tumors in humans and animals and follow-up observations after chemical treatment. Therefore, the target segment of the diagnostic, such as a living body, and more specifically a face, breast, cervix, and abdomen, is assumed as the subject 110.

A light absorbing body 120 which is the measurement object is preferably inside the subject 110 and has a relatively high light absorption coefficient. For example, when a human body is the measurement object, oxyhemoglobin or deoxyhemoglobin, blood vessels containing a large amount thereof, and malignant tumors containing a large number of new blood vessels become the light absorbing body which is the measurement object. Other examples include melanoma and plaque of the carotid artery wall.

### <Method for Measuring the Subject>

A method for measuring a living body, which is the subject, with the photoacoustic measurement method according to the present embodiment will be explained hereinbelow.

Where the living body is irradiated through the optical system 30 with the pulsed light emitted from the light source 10, part of the energy of the light propagating inside the living body reaches the light absorbing body 120 located inside the living body. The light absorbing body is typically a blood vessel, in particular a substance such as hemoglobin present inside the blood vessel, and a tumor. Where the light absorbing body absorbs the light energy, a photoacoustic wave is generated. The acoustic wave generated inside the living body propagates inside the subject and reaches the probe 20.

The acoustic wave received by the probe 20 is converted into time-series electric signals and successively inputted to the processing unit 60 for analysis. The analysis results are converted into image data representing the property information on the interior of the living body (for example, initial sound pressure distribution and absorption coefficient distribution) and outputted through the display unit 100. The image may be in the form, for example, of three-dimensional data or two-dimensional data.

Meanwhile, the irradiation light which has been diffused inside the subject 110 and emitted to the outside of the subject is detected by the first photodetector 40 and the intensity thereof is inputted to the processing unit 60. Part of the irradiation light with which the subject 110 has been irradiated is detected by the second photodetector 50 and the intensity thereof is inputted to the processing unit 60. In the present embodiment, the processing unit 60 determines the presence/absence of irradiation with the irradiation light and controls the light quantity on the basis of the intensity of light detected by the first photodetector 40 and the second photodetector 50.

### <Measurement Processing Flowchart>

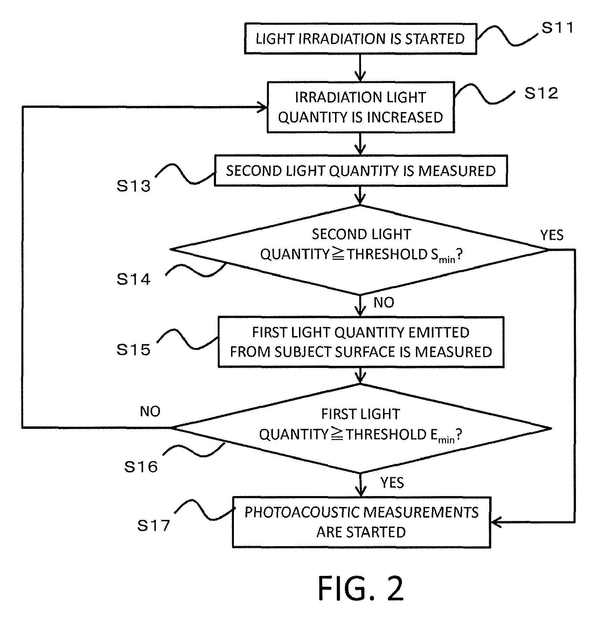

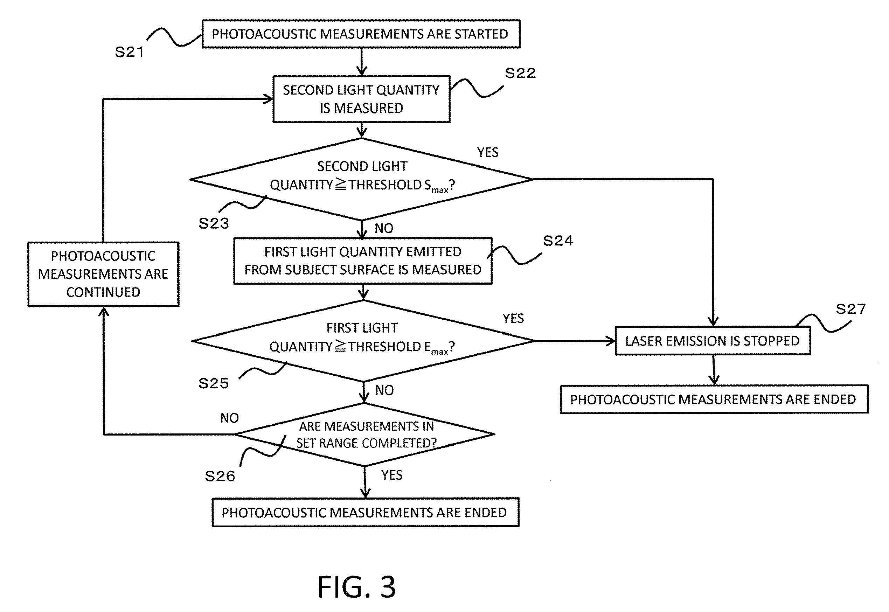

A more specific control method is explained hereinbelow with reference to FIGS. 2 and 3 which are the measurement processing flowcharts.

FIG. 2 is a flowchart illustrating the processing performed from after an operator instructs the device to start the measurements till the measurements are started.

Initially, in step S11, the control unit 80 instructs the light source 10 to start irradiation with the irradiation light. The light quantity of the irradiation light which is to be used for irradiation is sufficiently less than the maximum permissible exposure (MPE) which has been set for the subject. For example, when the irradiation segment is human skin, it is preferred that the quantity of light with which the surface of the subject 110 is irradiated be equal to or less than 1/10 the MPE which has been set for the skin.

Then, in step S12, the control unit 80 increases the quantity of the irradiation light by 1 step. The width of change of the light quantity may take any value, but is preferably not changed rapidly. For example, the width of change can be 1/10 of the MPE.

Then, in step S13, the processing unit 60 (second light quantity collection unit 63) acquires the signal outputted by the second photodetector 50 and converts the signal into a light quantity. The light quantity acquired herein may be further converted to determine the light quantity emitted per unit surface area of the subject 110.

The light quantity acquired by the second light quantity collection unit 63 is referred to hereinbelow as "second light quantity".

Then, in step S14, the processing unit 60 (light quantity determination unit 65) determines whether or not the second light quantity is larger than a preset threshold (the sixth threshold in the present invention; referred to hereinbelow as threshold Sₘᵢₙ). Where the second light quantity is greater than the threshold Sₘᵢₙ, subsequent increase in the light quantity is determined to be unnecessary and the processing advances to step S17. Where the second light quantity is not above the threshold Sₘᵢₙ, the processing advances to step S15.

The threshold Sₘᵢₙ used herein provides a safety margin with respect to the irradiation segment. For example, when the irradiation segment of the irradiation light is human skin, the threshold Sₘᵢₙ can be a half of the MPE which has been set for the skin. It goes without saying, that other values may be also used.

Where the second light quantity has not reached the threshold Sₘᵢₙ, the processing of step S15 is executed.

The processing of steps S15 and S16 serves to start the measurements even when the second light quantity has not reached the threshold Sₘᵢₙ when the quantity of light emitted from the surface of the subject is acquired and the light quantity is equal to or greater than a predetermined threshold.

In step S15, the processing unit 60 (first light quantity collection unit 62) acquires the signal outputted by the first photodetector 40 and converts the signal into a light quantity. The acquired light quantity may be further converted to determine the quantity of light, per unit surface area, incident on the first photodetector 40.

The light quantity acquired by the first light quantity collection unit 62 is referred to hereinbelow as "first light quantity".

Then, in step S16, the processing unit 60 (light quantity determination unit 65) determines whether or not the first light quantity is larger than a preset threshold (the fifth threshold in the present invention; referred to hereinbelow as threshold Eₘᵢₙ). Where the first light quantity is greater than the threshold Eₘᵢₙ, the processing advances to step S17 regardless of the second light quantity. Where the first light quantity is not above the threshold Eₘᵢₙ, the processing advances to step S12.

The threshold Eₘᵢₙ used herein provides a safety margin with respect to a segment for which the MPE is set more stringently than for the irradiation segment. For example, where the irradiation segment is close to human eyes, the threshold Eₘᵢₙ can be a half of the MPE which has been set for the eyes (retina). It goes without saying, that other values may be also used. The threshold Eₘᵢₙ is less than the threshold Sₘᵢₙ.

Finally, in step S17, the control unit 80 starts photoacoustic measurements on the subject.

Where such processing is executed, the quantity of light emitted from the light source 10 gradually rises till the first light quantity exceeds the threshold Eₘᵢₙ or till the second light quantity exceeds the threshold Sₘᵢₙ. As a result, it is possible to determine the irradiation light quantity providing a safety margin with respect to the segment irradiated with the irradiation light and also the segment which is close to the aforementioned segment and has a more stringent MPE.

The processing illustrated by FIG. 2 is preferably performed for each emission of the pulsed light, but may be also performed by thinning out every few pulses. It may also be performed every certain period of time.

Additionally, in the explanation of the embodiments with reference to FIG. 2, the photoacoustic measurement device executes the step of increasing the light quantity (step S12) at first, but the order of the process is not limited to the disclosed embodiments.

For example, after starting the light irradiation (step S11), the process may omit step S12. In this case, step S12 is processed after the first light quantity is determined to be less than the threshold Eₘᵢₙ (step S16-NO).

FIG. 3 is a flowchart illustrating the processing of controlling the quantity of light with which the subject is irradiated during the photoacoustic measurements.

Initially, in step S21, the photoacoustic measurements are started.

When, in step S22, the processing unit 60 (second light quantity collection unit 63) acquires the signal outputted by the second photodetector 50 and converts the signal into a light quantity in the same manner as in step S13. Since this processing is the same as that of step S13, detailed explanation thereof is herein omitted.

Then, in step S23, the processing unit 60 (light quantity determination unit 65) determines whether or not the second light quantity is larger than a preset threshold (the third threshold in the present invention; referred to hereinbelow as threshold Sₘₐₓ). Where the second light quantity is greater than the threshold Sₘₐₓ, it is determined that the light quantity is too large, the processing advances to step S27, the emission of the irradiation light is stopped, and the measurements are ended. Where the second light quantity is not above the threshold Sₘₐₓ, the processing advances to step S24.

The threshold Sₘₐₓ used herein is a restricted value of the irradiation light quantity with respect to the surface of the subject 110. For example, when the irradiation segment of the irradiation light is human skin, the threshold Sₘₐₓ can be two thirds of the MPE which has been set for the skin. It goes without saying, that other values may be also used.

In the present example, the emission of the irradiation light is stopped by transmitting a signal to stop the emission to the light source 10, but other methods may be used, provided that the irradiation of the subject with light can be stopped. For example, the light path may be cut off by using a laser shutter.

Where the second light quantity has not reached the threshold Sₘₐₓ, the processing of step S25 is executed.

The processing of steps S24 and S25 serves to stop the emission of the irradiation light even when the second light quantity has not reached the threshold Sₘₐₓ when the quantity of light emitted from the surface of the subject is equal to or greater than a predetermined threshold.

In step S24, the processing unit 60 (first light quantity collection unit 62) acquires the signal outputted by the first photodetector 40 and converts the signal into a light quantity in the same manner as in step S15. Since this processing is the same as that of step S15, detailed explanation thereof is herein omitted.

Then, in step S25, the processing unit 60 (light quantity determination unit 65) determines whether or not the first light quantity is larger than a preset threshold (the first threshold in the present invention; referred to hereinbelow as threshold Eₘₐₓ). Where the first light quantity is greater than the threshold Eₘₐₓ, the processing advances to step S27, regardless of the second light quantity, the emission of the irradiation light is stopped, and the measurements are ended. Where the first light quantity is not above the threshold Eₘₐₓ, the processing advances to step S26.

The threshold Eₘₐₓ used herein is a restricted value of the irradiation light quantity with respect to a segment for which the MPE is set more stringently than for the irradiation segment. For example, where the irradiation segment is close to human eyes, the threshold Eₘₐₓ can be two thirds of the MPE which has been set for the eyes (retina). It goes without saying, that other values may be also used, but the threshold Eₘₐₓ is less than the threshold Sₘₐₓ.

Finally, in step S26, it is determined whether the measurements for the desired range have been completed, and where the measurements have been completed, the photoacoustic measurements are ended. Where the measurements have not been completed, the processing is continued by performing the photoacoustic measurements with respect to the next measurement point.

Where such processing is executed, it is possible to detect that the first light quantity has exceeded the threshold Eₘₐₓ or that the second light quantity has exceeded the threshold Sₘₐₓ and to stop the irradiation of the subject with light. As a result, it is possible to perform monitoring such that the restricted value of light quantity is not exceeded with respect to both the segment irradiated with the irradiation light and also the segment which is close to the aforementioned segment and has a more stringent MPE.

The processing illustrated by FIG. 3 is preferably performed for each emission of the pulsed light, but may be also performed by thinning out every few pulses. It may also be performed every certain period of time.

As explained hereinabove, with the photoacoustic measurement device according to the present embodiment, the photoacoustic measurements can be performed with consideration not only for the safety of the living tissue (for example, skin) located in the irradiation segment, but also the safety of other light tissue (for example, retina) with respect to the diffused light propagating inside the subject.

Further, in the present embodiment, the processing depicted in FIG. 2 and the processing depicted in FIG. 3 are performed continuously, but it is not always necessary to perform the processing depicted in FIG. 2 prior to the measurements. For example, it is possible to carry out the irradiation with light at a predetermined light quantity which has been set in advance and immediately start the photoacoustic measurements. In this case, the predetermined light quantity is preferably set such as to be equal to or less than half of the MPE which has been set for the irradiation segment (that is, the skin).

### (Second Embodiment)

In the first embodiment, the emission of the irradiation light is stopped when the first light quantity is greater than the threshold Eₘₐₓ or the second light quantity is greater than the threshold Sₘₐₓ. By contrast, in the second embodiment, the control of reducing the irradiation light quantity is performed without immediately stopping the emission.

Since the configuration of the photoacoustic measurement device according to the second embodiment is the same as in the first embodiment, detailed explanation thereof is herein omitted and only the difference in processing is explained.

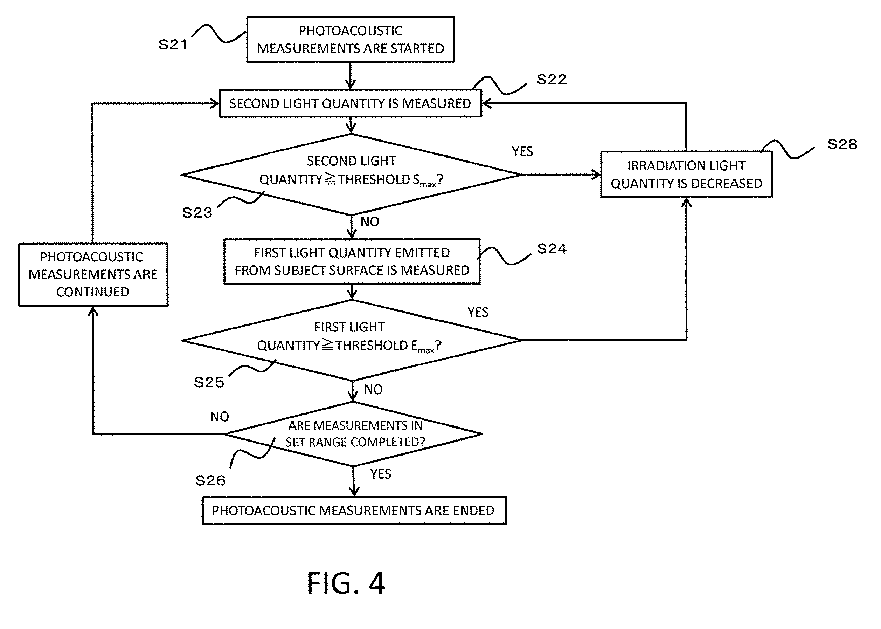

FIG. 4 is a flowchart illustrating the processing of controlling the quantity of light with which the subject is irradiated in the second embodiment. In the second embodiment, where it is determined in step S23 that the second light quantity exceeds the threshold Sₘₐₓ, the processing advances to step S28 and a command to decrease the irradiation light quantity is issued to the light source 10. Likewise, where it is determined that the first light quantity exceeds the threshold Eₘₐₓ, the processing advances to step S28 and a command to decrease the irradiation light quantity is issued to the light source 10. Other processing is the same as in the first embodiment.

According to the second embodiment, the measurements can be continued even when the first light quantity or second light quantity changes for some reason.

### (Third Embodiment)

In the second embodiment, the quantity of the irradiation light is decreased when the first light quantity is greater than the threshold Eₘₐₓ or the second light quantity is greater than the threshold Sₘₐₓ. By contrast in the third embodiment, the quantity of the irradiation light is increased again when the first and second light quantities are decreased after decreasing the quantity of the irradiation light.

Since the configuration of the photoacoustic measurement device according to the second embodiment is the same as in the first embodiment, detailed explanation thereof is herein omitted and only the difference in processing with the second embodiment is explained.

In the third embodiment, step S231 is executed after step S23 has been executed.

In step S231, the processing unit 60 (second light quantity collection unit 63) determines whether or not the second light quantity is larger than a preset threshold (the fourth threshold in the present invention; here, the threshold Sₘᵢₙ which is the same value as the sixth threshold). Where the second light quantity is less than the threshold Sₘᵢₙ, it is determined that the light quantity has decreased too much, the processing advances to step S29, and the quantity of the irradiation light is increased. Where the second light quantity is not less than the threshold Sₘᵢₙ, the processing advances to step S24.

In the same manner as in the first embodiment, the threshold Sₘᵢₙ used herein is less than the threshold Sₘₐₓ. For example, when the threshold Sₘₐₓ is two thirds of the MPE which has been set for the skin, the threshold Sₘᵢₙ can be a half of the MPE which has been set for the skin.

Further, in the third embodiment, step S251 is executed after step S25 has been executed.

In step S251, the processing unit 60 (first light quantity collection unit 62) determines whether or not the first light quantity is larger than a preset threshold (the second threshold in the present invention; here, the threshold Eₘᵢₙ which is the same value as the fifth threshold). Where the first light quantity is less than the threshold Eₘᵢₙ, it is determined that the light quantity has decreased too much, the processing advances to step S29, and the quantity of the irradiation light is increased. Where the first light quantity is not less than the threshold, the processing advances to step S26.

In the same manner as in the first embodiment, the threshold Eₘᵢₙ used herein is less than the threshold Eₘₐₓ. For example, when the threshold Eₘₐₓ is two thirds of the MPE which has been set for the eye (retina), the threshold Eₘᵢₙ can be a half of the MPE which has been set for the eyes.

Other processing is the same as in the first embodiment

According to the third embodiment, the quantity of the irradiation light can be maintained in an adequate range, that is, a range suitable for measurements which provides a safety margin.

### (Variation Examples)

The embodiments described hereinabove merely illustrate the present invention, and the implementation of the present invention can involve appropriate changes and combination, without departing from the essence of the invention.

For example, the present invention can be also implemented as a photoacoustic device including at least some of the above-described units. It can be also implemented as a control method for the photoacoustic device. The above-describes processing operations and units can be freely combined together, provided that no technical contradiction is created thereby.

Further, the present invention can be also implemented as a hand-held photoacoustic device. In this case, a configuration may be used in which the first photodetector 40 is accommodated in addition to the measurement unit 140 in an integrated probe.

In the description of the embodiments, four thresholds for light quantity are presented, namely, Sₘᵢₙ, Sₘₐₓ, Eₘᵢₙ, and Eₘₐₓ, but values other than those presented by way of example may be also used as the thresholds. Further, the second threshold and fifth threshold, and the fourth threshold and sixth threshold may have different values.

For example, it is preferred that the first threshold be equal to or less than two thirds of the MPE which has been set for the eyes, and the second and fifth thresholds be equal to or greater than a half of the MPE which has been set for the eyes, but other values may be also used. Likewise, it is preferred that the third threshold be equal to or less than two thirds of the MPE which has been set for the skin and the fourth and sixth thresholds be equal to or greater than a half of the MPE which has been set for the skin, but other values may be also used.

Further, in the description of the embodiment, the irradiation control based on the light quantity determination results is performed automatically by the processing unit 60 and the control unit 80, but the irradiation control may be performed manually. In this case, the determination results may be outputted to the display unit 100, and the operator observing the outputted determination results may determine whether or not to perform the irradiation with the irradiation light manually or to adjust the light quantity.

### (Other Embodiments)

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

A photoacoustic device comprises a light irradiation unit configured to irradiate a subject with light; an acoustic wave detector configured to detect an acoustic wave generated inside the subject and convert the detected acoustic wave into an electric signal; a first photodetector configured to acquire information relating to an intensity of light that has propagated inside the subject and leaked to the outside of the subject; an information acquisition unit configured to acquire information on an interior of the subject on the basis of the electric signal; and a control unit configured to control the light with which the subject is irradiated on the basis of the information relating to the intensity of light which has been acquired by the first photodetector.

## Claims

1. A photoacoustic device comprising:
a light irradiation unit configured to irradiate a subject with light;
an acoustic wave detector configured to detect an acoustic wave generated inside the subject under the irradiation with light and convert the detected acoustic wave into an electric signal;
a first photodetector configured to acquire information relating to an intensity of light that has propagated inside the subject and leaked to the outside of the subject;
an information acquisition unit configured to acquire information on an interior of the subject on the basis of the electric signal; and
a control unit configured to control the light with which the subject is irradiated on the basis of the information relating to the intensity of light which has been acquired by the first photodetector.

2. The photoacoustic device according to claim 1, wherein
the first photodetector is in intimate contact with a surface of the subject.

3. The photoacoustic device according to claim 1 or 2, wherein
the control unit stops the irradiation of the subject with light when the intensity of light acquired by the first photodetector is greater than a first threshold.

4. The photoacoustic device according to claim 1 or 2, wherein
the control unit decreases the intensity of light with which the subject is irradiated when the intensity of light acquired by the first photodetector is greater than a first threshold.

5. The photoacoustic device according to claim 3 or 4, wherein
the control unit increases the intensity of light with which the subject is irradiated when the intensity of light acquired by the first photodetector is less than a second threshold.

6. The photoacoustic device according to claim 5, wherein
the first threshold and the second threshold are set on the basis of a maximum permissible exposure with respect to eyes.

7. The photoacoustic device according to claim 6, wherein
the first threshold is a value that is less than two thirds of the maximum permissible exposure with respect to eyes, and
the second threshold is a value that is greater than half of the maximum permissible exposure with respect to eyes.

8. The photoacoustic device according to any one of the claims 1 to 7, further comprising:
a second photodetector configured to acquire the intensity of light with which the subject is irradiated, wherein
the control unit controls the intensity of light with which the subject is irradiated also on the basis of the intensity of light acquired by the second photodetector.

9. The photoacoustic device according to claim 8, wherein
the control unit stops the irradiation of the subject with light when the intensity of light acquired by the second photodetector is greater than a third threshold.

10. The photoacoustic device according to claim 8, wherein
the control unit decreases the intensity of light with which the subject is irradiated when the intensity of light acquired by the second photodetector is greater than a third threshold.

11. The photoacoustic device according to claim 9 or 10, wherein
the control unit increases the intensity of light with which the subject is irradiated when the intensity of light acquired by the second photodetector is less than a fourth threshold.

12. The photoacoustic device according to any one of the claims 9 to 11, wherein
the control unit:
controls timings of measurements performed on the subject by controlling operation of each of the light irradiation unit, acoustic wave detector, and information acquisition unit;
gradually increases the intensity of light when starting the irradiation of the subject with light; and
starts the measurements performed on the subject when the intensity of light acquired by the first photodetector is equal to or greater than a fifth threshold that is less than the first threshold, or when the intensity of light acquired by the second photodetector is equal to or greater than a sixth threshold that is less than the third threshold.

13. The photoacoustic device according to claim 11, wherein
the third threshold and the fourth threshold are set on the basis of a maximum permissible exposure with respect to skin.

14. A control method for a photoacoustic device including a light source configured to irradiate a subject with light, and an acoustic wave detector configured to detect an acoustic wave generated inside the subject under the irradiation with light, the method comprising:
an acoustic wave detection step of converting the acoustic wave generated inside the subject due to the light, into an electric signal;
a first light detection step of acquiring information relating to an intensity of light that has propagated inside the subject and leaked to the outside of the subject;
an information acquisition step of acquiring information on an interior of the subject on the basis of the electric signal; and
a control step of controlling the light with which the subject is irradiated on the basis of the information relating to the intensity of light which has been acquired in the first light detection step.

15. A non-transitory computer readable storing medium recording a computer program for causing a computer to perform a control method for a photoacoustic device including a light source configured to irradiate a subject with light, and an acoustic wave detector configured to detect an acoustic wave generated inside the subject under the irradiation with light, the method comprising:
an acoustic wave detection step of converting the acoustic wave generated inside the subject due to the light, into an electric signal;
a first light detection step of acquiring information relating to an intensity of light that has propagated inside the subject and leaked to the outside of the subject;
an information acquisition step of acquiring information on an interior of the subject on the basis of the electric signal; and
a control step of controlling the light with which the subject is irradiated on the basis of the information relating to the intensity of light which has been acquired in the first light detection step.
